# EUROPEAN PATENT APPLICATION

(11) **EP 4 327 659 A2**
(43) Date of publication of application: **28.02.2024**
(21) Application number: 23193483.7
(22) Date of filing: 25.08.2023
(51) Int. Cl.: A01N 63/20, C05F 11/08, A01N 63/27, C12N 1/20, A01P 21/00, C12R 1/01

(54) **MICROBIAL INOCULANTS AND METHODS**

(30) Priority: 25.08.2022 US 202263400799 P; 17.03.2023 US 202363452849 P
(71) Applicant: Ceragen Inc., Kitchener, ON N2G 1H6 (CA)
(72) Inventor: ROSE, Danielle, WATERLOO (CA); STEGELMEIER, Ashley, KITCHENER (CA); ROSE, Matthew, WATERLOO (CA); JORIS, Ben, KITCHENER (CA); SERRAVALLE, Erika, KITCHENER (CA); DRAPEAU, Michelle, KITCHENER (CA)
(74) Representative: Algemeen Octrooi- en Merkenbureau B.V.

(57) **Abstract**

In the present invention, microbial inoculants that promoted leafy green plant growth were developed using *Rhodococcus erythropolis, Stenotrophomonas maltophilia, Serratia grimesii,* and *Pseudomonas toyotomiensis.* The microbial inoculants include probiotics effective on their own and others effective in specific combinations as follows: *Rhodococcus erythropolis; Rhodococcus erythropolis* and *Serratia grimesii; Serratia grimesii; Serratia grimesii* and *Pseudomonas toyotomiensis; Pseudomonas toyotomiensis* and *Stenotrophomonas maltophilia*; *Rhodococcus erythropolis, Pseudomonas toyotomiensis* and *Stenotrophomonas maltophilia*; *Rhodococcus erythropolis, Pseudomonas toyotomiensis, Stenotrophomonas maltophilia,* and *Serratia grimesii; Shinella zoogloeoides;* and *Rhodococcus erythropolis, Pseudomonas toyotomiensis, Stenotrophomonas maltophilia, Serratia grimesii* and *Shinella zoogloeoides.* It was found that other probiotics were not effective on their own and there were other combinations that were not effective or inhibitory for plant growth.

## Description

### Field of the Invention

The present invention relates to microbial inoculants and more specifically to microbial inoculants that promoted leafy green plant growth either singly or in specific combinations.

### Background of the Invention

The plant microbiome is the community of bacteria, archaea, fungi, and viruses that live on, around, and inside of a plant.¹ Microbes play an important role in the health and wellbeing of plants, including helping plants respond better to environmental stresses.² These microbes can help provide nutrients for the plant, promote plant growth, and regulate hormone levels. For some plants it is known that microbial inoculants can increase crop yields by reducing negative stress effects such as leaf yellowing, leaf loss, and impaired plant growth. Microbial inoculants have also been shown to increase crop yields by improving the uptake or availability to the plant of nutrients, such as iron or phosphorus.³

Microbial inoculants are compositions comprising beneficial microbes that promote plant health. Microbial inoculants can help farmers significantly increase crop yields. However, bacterial species are specific to the type of plants that they can interact with,⁴⁻⁵ and many microbes cannot make the transition to soilless based growing systems. The nutritional composition of the plant root exudates contains a complex mixture of sugars, amino acids, hormones, antimicrobials, and vitamins.¹ The variation in root exudates between plants is one of the main drivers that determines which microorganisms can associate with the roots of each species.¹ By extension, previous research has determined that root colonization capacity does not predict the ability of an organism to survive in hydroponics systems.⁶ A study by Politz *et al* concluded that "In general, *in vitro* assays were not useful predictors of the relative performance of isolates *in planta*", likely due to their methodology of using microorganisms obtained from soils to inoculate cucumbers in a hydroponics environment.⁷ When soil was added into a hydroponics system containing maize seedlings, a community shift in the types of organisms that can effectively survive on the roots was observed.⁸ The successful root colonizers were predominately shorter rods with simpler nutritional demands compared to the microbial community found in soil.

To date, research on plant growth promoting bacteria in lettuce has focused primarily on soil-based studies. Numerous researchers have demonstrated that meaningful yield increases in soil-based lettuce agriculture can be achieved using microbial inoculants.⁹⁻¹⁸ However, fewer studies have specifically focused on hydroponics lettuce research.¹⁹⁻²⁶ These studies provided the foundation that microbial inoculants can be effective in lettuce. However, their conclusions were often based on relatively small numbers of plant replicates, few candidate bacterial strains, or only tested a single bacterial strain instead of a consortium. There is currently no commercially available microbial inoculant designed and optimized specifically for hydroponic leafy greens.

### References

1. Turner, T.R., James, E.K., & Poole, P.S. (2013). The plant microbiome. Genome Biology, 14:209.
2. Glick, B.R. (2014). Bacteria with ACC deaminase can promote plant growth and help to feed the world. Microbiological Research, 169(1): 30-39.
3. Sammauria, R., Kumawat, S., Kumawat, P., Singh, J., & Jatwa, T.K. (2020). Microbial inoculants: potential tool for sustainability of agricultural production systems. Archives of Microbiology, 202: 677-693.
4. Drogue, B., Doré H., Borland, S., Wisniewski-Dyé, & F. Prigent-Combaret, C. (2012). Which specificity in cooperation between phytostimulating rhizobacteria and plants? Research in Microbiology, 163(8): 500-510.
5. Sanchis-López, C., Cerna-Vargas, J.P., Santamaria-Hernando, S., Ramos, C., Krell, T., Rodriguez-Palenzuela, P., López-Solanilla, E., Huerta-Cepas, J., & Rodríguez-Hervaa, J.J. (2021). Prevalence and specificity of chemoreceptor profiles in plant associated bacteria. mSystems, 6(5): e00951-21.
6. Moralles, A., Garland, J.L., & Lim, D.V. (1996). Survival of potentially pathogenic human-associated bacteria in the rhizosphere of hydroponically grown wheat. FEMS Microbiology Ecology, 20(1996): 155-162.
7. Paulitz, T.C., Zhou, T., & Rankin, L. (1992). Selection of rhizosphere bacteria for biological control of Pythium aphanidermatum on hydroponically grown cucumber. Biological Control, 2(3): 226-237.
8. Schönwitz, R., & Ziegler, H. (1986). Quantitative and qualitative aspects of a developing rhizosphere microflora of hydroponically grown maize seedling. Journal of Plant Nutrition and Soil Science, 149: 623-634.
9. Sahin, U., Ekinci, M., Kiziloglu, F.M., Yildirim, E., Turan, M., Kotan, R., & Ors, S. (2015). Ameliorative effects of plant growth promoting bacteria on water-yield relationships, growth, and nutrient uptake of lettuce plants under different irrigation levels. Hortscience, 50(9): 1379-1386.
10. Cipriano., M.A.P., Lupatini, M., Lopes-Santos, L., da Silva, M.J., Roesch, L.F.W., Destefano, S.A.L., Freitas, S.S., & Kuramae, E.E. (2016). Lettuce and rhizosphere microbiome responses to growth promoting Pseudomonas species under field conditions. FEMS Microbiology Ecology, 92(12): fiw 197.
11. Szczech, M., Szafirowska, A., Kowalczyk, W., Szwejda-Grzybowska, J., Wlodarek, A., & Maciorowski, R. (2016). The effect of plant growth promoting bacteria on transplants growth and lettuce yield in organic production. Journal of Horticultural Research, 24(2): 101-107.
12. Ferreira, J.T.P., Santos, T.M.C., Albuquerque, L.S., Santos, J.V., Cardoso Filho, J.A.C., & Ramalho Neto, C.E. (2011). Isolation and selection of growth-promoting bacteria of the genus Bacillus and its effect on two varieties of lettuce (Lactuca sativa L.). International Research Journal of Microbiology, 2(2): 070-078.
13. Kang, S.M., Khan, A.L., Hussain, J., Ali, L., Kamran, M., Waqas, M., & Lee, I.J. (2012). Rhizonin A from Burkholderia sp. KCTC11096 and its growth promoting role in lettuce seed germination. Molecules, 17(7): 7980-7988.
14. Vetrano, F., Miceli, C., Angileri, V., Frangipane, B., Moncada, A., & Miceli, A. (2020). Effect of bacterial inoculum and fertigation management on nursery and field production of lettuce plants. Agronomy, 10(10): 1477.
15. Sottero, A.N., dos Santos Freitas, S., Tavares de Melo., A.M., & Trani, P.E. (2006). Rhizobacteria and lettuce: root colonization, plant growth promotion and biological control. Revista Brasileira de Ciência do Solo, 30(2).
16. Verma, M., Singh, A., Dwivedi, D.H., & Arora, N.K. (2020). Zinc and phosphate solubilizing Rhizobium radiobacter (LB2) for enhancing quality and yield of loose leaf lettuce in saline soil. Environmental Sustainability, 3: 209-218.
17. Ahmadi, N., Fatemi, H., Esmaielpour, B., & Soultani-Tolarood, A.A.(2020). Effect of bio-priming with plant growth promoting bacteria on growth and biochemical characteristics, phenol, flavonoid, vitamin C and nitrate in lettuce (Lactuca sativa L.) Rabicon cultivar in different growth substrates. Journal of Science and Technology of Greenhouse Culture, 11(2): fa41-fa58.
18. Kohler, J., Caravaca, F., Carrasco, L., & Roldan, A.(2006). Contribution of Pseudomonas mendocina and Glomus intraradices to aggregate stabilization and promotion of biological fertility in rhizosphere soil of lettuce plants under field conditions. Soil Use and Management, 22: 298-304.
19. Moncada, A., Vetrano, F., & Miceli, A. (2020). Alleviation of salt stress by plant growth-promoting bacteria in hydroponic leaf lettuce. Agronomy, 2020(10): 1523.
20. Aini, N., Yamika, W.S.D., & Ulum, B. (2019). Effect of nutrient concentration, PGPR and AMF on plant growth, yield and nutrient uptake of hydroponic lettuce. International Journal of Agriculture and Biology, 21: 175-183.
21. Utkhede, R.S., Lévesque, C.A., & Dinh, D. (2000). Pythium aphanidermatum root rot in hydroponically grown lettuce and the effect of chemical and biological agents on its control. Canadian Journal of Plant Pathology, 22:2: 138-144.
22. Sebring, R.L. Duiker, S.W. Berghage, R.D. Regan, J.M. Lambert, J.D. & Bryant, R.B. (2022). Gluconacetobacter diazotrophicus inoculation of two lettuce cultivars affects leaf and root growth under hydroponic conditions. Applied Sciences, 12: 1585.
23. Riser, E.C., Grabowski, J., & Glenn, E.P. (1984). Microbiology of hydroponically-grown lettuce. Journal of Food Protection, 47(10): 765-769.
24. De Almeida Moreira, V., da Silva Oliveira, C.E., Jalal, A., Bueno Gato, I.M., Sena Oliveira, T.J.S.S., Boleta, G.H.M., & Giolo, V.M. (2022). Inoculation with Trichoderma harzianum and Azospirillum brasilense increases nutrition and yield of hydroponic lettuce. Archives of Microbiology, 204: 440.
25. Ajijah, N., Apriyana, A.Y., Sriwuryandari, L., Priantoro, E.A., Janetasari, S.A., Pertiwi, T.Y.R., Suciati, A.M., Ardeniswan, & Sembiring, T. (2021). Beneficiary of nitrifying bacteria for enhancing lettuce (Lactuca sativa) and vetiver grass (Chrysopogon zizanioides L.) growths align with carp (Cyprinus carpio) cultivation in an aquaponic system. Environmental Science and Pollution Research, 28: 880-889.
26. Suzuki, W., Sugawara, M., Miwa, K., & Morikawa, M. (2014). Plant growth-promoting bacterium Acinetobacter calcoaceticus P23 increases the chlorophyll content of the monocot Lemna minor (duckweed) and the dicot Lactuca sativa (lettuce). Journal of Bioscience and Bioengineering, 118(1): 41-44.

### Summary of the Invention

In a first aspect, the present invention relates to a microbial inoculant for promoting leafy green plant growth comprising the bacterial species:
i) *Rhodococcus erythropoli,* optionally in combination with *Serratia grimesia*,
ii) *Serratia grimesia*, optionally in combination with *Pseudomonas toyotomiensis,*
iii) *Shinella zoogloeoides,* or
iv) *Pseudomonas toyotomiensis* in combination *Stenotrophomonas maltophilia*, and optionally further in combination with and *Rhodococcus erythropoli* and optionally also *Serratia grimesia* and further optionally also in combination with *Shinella zoogloeoides.*

The present invention is a microbial inoculant for promoting leafy green plant growth comprising *Rhodococcus erythropolis.*

The present invention is a microbial inoculant for promoting leafy green plant growth comprising *Rhodococcus erythropolis* with a 98% nucleotide sequence similarity to the 16S region of SEQ ID NO: 1.

The present invention is a microbial inoculant for promoting leafy green plant growth comprising *Rhodococcus erythropolis* and *Serratia grimesii.*

The present invention is a microbial inoculant for promoting leafy green plant growth comprising *Serratia grimesii.*

The present invention is a microbial inoculant for promoting leafy green plant growth comprising *Serratia grimesii* with a 98% nucleotide sequence similarity to the 16S region of SEQ ID NO:2.

The present invention is a microbial inoculant for promoting leafy green plant growth comprising *Serratia grimesii* with a 98% nucleotide sequence similarity to the 16S region of SEQ ID NO:2 and *Rhodococcus erythropolis* with a 98% nucleotide sequence similarity to the 16S region of SEQ ID NO: 1.

The present invention is a microbial inoculant for promoting leafy green plant growth comprising *Serratia grimesii* and *Pseudomonas toyotomiensis.*

The present invention is a microbial inoculant for promoting leafy green plant growth comprising *Serratia grimesii* with a 98% nucleotide sequence similarity to the 16S region of SEQ ID NO:2 and *Pseudomonas toyotomiensis* with a 98% nucleotide sequence similarity to the 16S region of SEQ ID NO:4.

The present invention is a microbial inoculant for promoting leafy green plant growth comprising *Stenotrophomonas maltophilia* and *Pseudomonas toyotomiensis.*

The present invention is a microbial inoculant for promoting leafy green plant growth comprising *Stenotrophomonas maltophilia* with a 98% nucleotide sequence similarity to the 16S region of SEQ ID NO:3 and *Pseudomonas toyotomiensis* with a 98% nucleotide sequence similarity to the 16S region of SEQ ID NO:4.

The present invention is a microbial inoculant for promoting leafy green plant growth comprising *Stenotrophomonas maltophilia* and *Pseudomonas toyotomiensis* and *Rhodococcus erythropolis.*

The present invention is a microbial inoculant for promoting leafy green plant growth comprising *Stenotrophomonas maltophilia* with a 98% nucleotide sequence similarity to the 16S region of SEQ ID NO:3 and *Pseudomonas toyotomiensis* with a 98% nucleotide sequence similarity to the 16S region of SEQ ID NO:4 and *Rhodococcus erythropolis* with a 98% nucleotide sequence similarity to the 16S region of SEQ ID NO: 1.

The present invention is a microbial inoculant for promoting leafy green plant growth comprising *Stenotrophomonas maltophilia* and *Pseudomonas toyotomiensis* and *Rhodococcus erythropolis* and *Serratia grimesii.*

The present invention is a microbial inoculant for promoting leafy green plant growth comprising *Stenotrophomonas maltophilia* with a 98% nucleotide sequence similarity to the 16S region of SEQ ID NO:3 and *Pseudomonas toyotomiensis* with a 98% nucleotide sequence similarity to the 16S region of SEQ ID NO:4 and *Rhodococcus erythropolis* with a 98% nucleotide sequence similarity to the 16S region of SEQ ID NO:1 and *Serratia grimesii* with a 98% nucleotide sequence similarity to the 16S region of SEQ ID NO:2.

The present invention is a microbial inoculant for promoting leafy green plant growth comprising *Shinella zoogloeoides.*

The present invention is a microbial inoculant for promoting leafy green plant growth comprising *Shinella zoogloeoides* with a 98% nucleotide sequence similarity to the 16S region of SEQ ID NO:5.

The present invention is a microbial inoculant for promoting leafy green plant growth comprising *Stenotrophomonas maltophilia* and *Pseudomonas toyotomiensis* and *Rhodococcus erythropolis* and *Serratia grimesii* and *Shinella zoogloeoides.*

The present invention is a microbial inoculant for promoting leafy green plant growth comprising *Stenotrophomonas maltophilia* with a 98% nucleotide sequence similarity to the 16S region of SEQ ID NO:3 and *Pseudomonas toyotomiensis* with a 98% nucleotide sequence similarity to the 16S region of SEQ ID NO:4 and *Rhodococcus erythropolis* with a 98% nucleotide sequence similarity to the 16S region of SEQ ID NO:1 and *Serratia grimesii* with a 98% nucleotide sequence similarity to the 16S region of SEQ ID NO:2 and *Shinella zoogloeoides* with a 98% nucleotide sequence similarity to the 16S region of SEQ ID NO:5.

The present invention is any one of the above microbial inoculants in which the leafy green plant is *Lactuca sativa.*

The present invention is the use of a composition comprising the microbial inoculant, the inoculant as broadly defined herein, promoting plant growth. Preferably, the use comprises inoculating the roots of leafy green plants, directly or through a water supply, with the inoculant as broadly defined herein. The present invention is the use of any one of the above microbial inoculants in one of the growing systems of hydroponic, aeroponic, soilless or soil based.

The present invention is a method for promoting plant growth comprising applying a composition of any one of the above microbial inoculants directly or through a water supply to the roots of the leafy green plants in an amount that provides for positive benefits relative to a control leafy green plant that does not receive an application of said microbial inoculant.

### Brief Description of the Figures

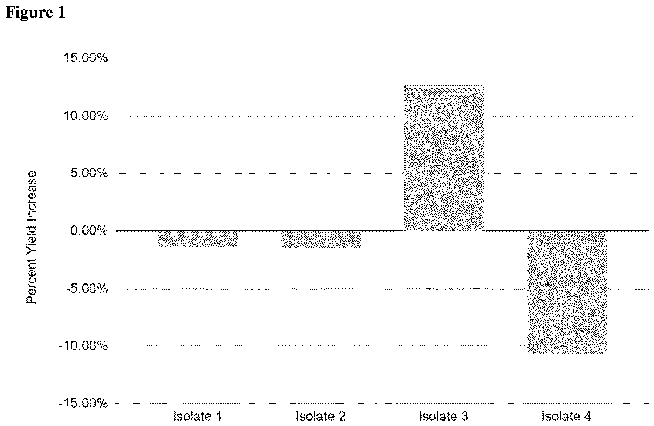

Figure 1 is a chart comparing the average percent yield increases of hydroponic lettuce that received a microbial inoculant of *Rhodococcus erythropolis* compared to control plants that received no inoculant, in a first embodiment of the present invention versus other inoculants.

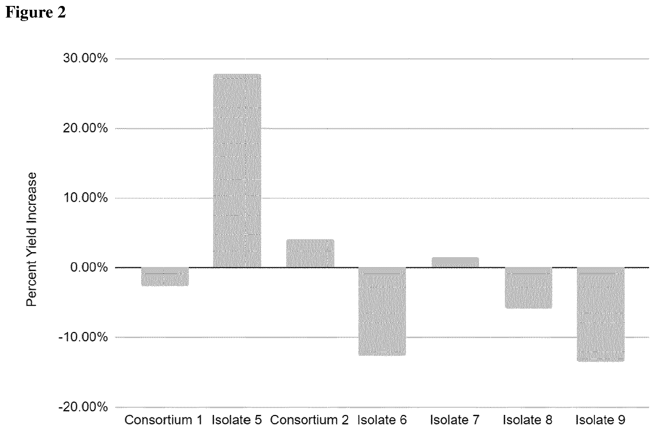

Figure 2 is a chart comparing the average percent yield increases of hydroponic lettuce that received a microbial inoculant of *Serratia grimesii* in a second embodiment of the present invention, and a microbial inoculant of a combination of *Pseudomonas toyotomiensis* and *Serratia grimesii* in a third embodiment of the present invention, versus control plants that received no inoculant.

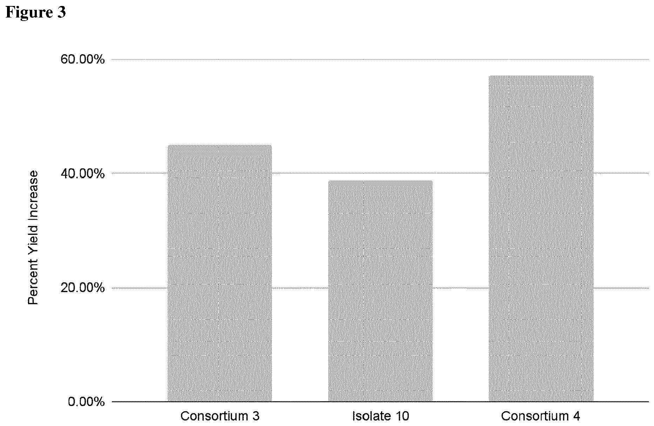

Figure 3 is a chart comparing the average percent yield increases of hydroponic lettuce that received a microbial inoculant of the second embodiment of the invention of *Serratia grimesii,* and a fourth embodiment of the present invention of a combination of *Stenotrophomonas maltophilia* and *Pseudomonas toyotomiensis,* and a fifth embodiment of the present invention of a combination of *Serratia grimesii* and *Rhodococcus erythropolis,* compared to control plants that received no inoculant.

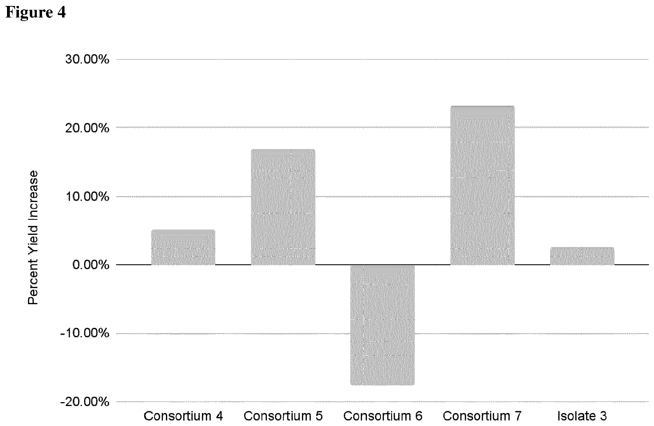

Figure 4 is a chart comparing the average percent yield increases of hydroponics lettuce that received a microbial inoculant of the first embodiment of the present invention of *Rhodococcus erythropolis,* the fifth embodiment of the present invention of a combination of *Serratia grimesii* and *Rhodococcus erythropolis*, a sixth embodiment of the present invention of *Rhodococcus erythropolis*, *Pseudomonas toyotomiensis,* and *Stenotrophomonas maltophilia*, and a seventh embodiment of the present invention of *Rhodococcus erythropolis*, *Serratia grimesii*, *Stenotrophomonas maltophilia*, and *Pseudomonas toyotomiensis,* compared to control plants that received no inoculant.

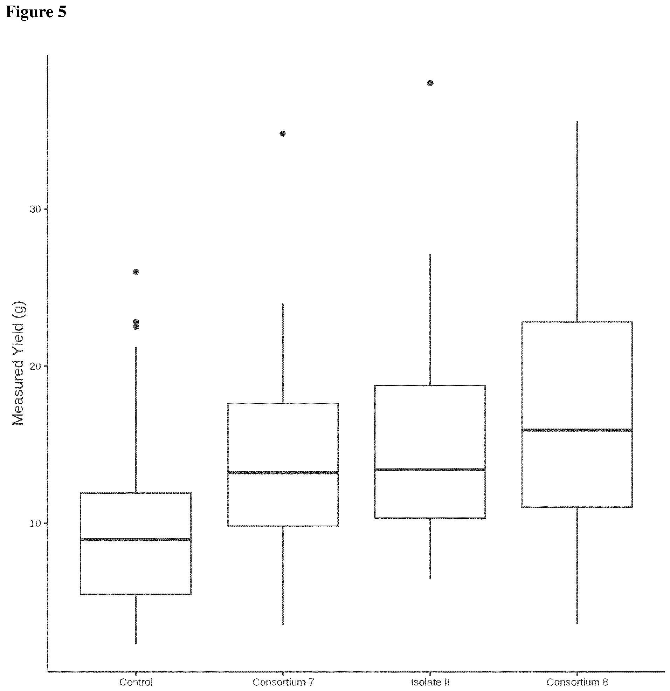

Figure 5 is a box plot of the average fresh lettuce yields for a hydroponic lettuce inoculant trial comparing the control plants which received no microbial inoculant to the seventh embodiment of the present invention (Consortium 7) which is a combination of *Rhodococcus erythropolis, Serratia grimesii, Stenotrophomonas maltophilia*, and *Pseudomonas toyotomiensis,* an eighth embodiment of the present invention (Isolate 11) which is a pure culture of *Shinella zoogloeoides*, and a ninth embodiment of the present invention (Consortium 8) which is a combination of *Rhodococcus erythropolis, Serratia grimesii, Stenotrophomonas maltophilia, Pseudomonas toyotomiensis,* and *Shinella zoogloeoides.*

### Detailed Description of the Invention

The present invention is a microbial inoculant for increasing leafy green growth. In a first embodiment of the present invention, the microbial inoculant comprises *Rhodococcus erythropolis.*

As set out in Figure 1, Isolate 3 is a pure culture of *Rhodococcus erythropolis.* Figure 1 shows the average percent yield increases for a hydroponic lettuce inoculant trial in comparison to the control plants that received no inoculant. Isolate 1 comprises a pure culture of *Pseudomonas putida.* Isolate 2 comprises a pure culture of *Curvibacter putative.* Isolate 4 comprises a pure culture of *Xylophilus rhododendri.* The microbial inoculant of *Rhodococcus erythropolis* is the only one to demonstrate increased yields in hydroponic lettuce. The increased yields from a microbial inoculant of *Rhodococcus erythropolis* is also demonstrated in Figure 4.

In a second embodiment of the present invention, the microbial inoculant is *Serratia grimesii.* In a third embodiment of the present invention, the microbial inoculant is a combination of *Pseudomonas toyotomiensis* and *Serratia grimesii.*

As set out in Figure 2, Isolate 5 comprises a pure culture of *Serratia grimesii* and Consortium 2 comprises a combination of *Pseudomonas toyotomiensis* and *Serratia grimesii.* Figure 2 shows the average percent yield increases for a hydroponic lettuce inoculant trial in comparison to the control plants that received no inoculant. Consortium 1 comprises a combination of *Stenotrophomonas maltophilia* and *Serratia grimesii.* Isolate 6 comprises a pure culture of *Pseudomonas rhizophila.* Isolate 7 is a pure culture of *Pseudomonas zarinae.* Isolate 8 is a pure culture of *Pseudarthrobacter psychrotolerans.* Isolate 9 comprises a pure culture of *Paenarthrobacter aurescens.* The microbial inoculant comprising *Serratia grimesii* and the microbial inoculant comprising the combination of *Pseudomonas toyotomiensis* and *Serratia grimesii* are the only ones to demonstrate significantly increased yields in hydroponic lettuce. The increased yields from a microbial inoculant of *Serratia grimesii* is also demonstrated in Figure 3.

In a fourth embodiment of the present invention, the microbial inoculant is a combination of *Stenotrophomonas maltophilia* and *Pseudomonas toyotomiensis.* In a fifth embodiment of the present invention, the microbial inoculant is a combination of *Serratia grimesii* and *Rhodococcus erythropolis.*

Figure 3 shows the average percent yield increases for a hydroponic lettuce inoculant trial in comparison to the control plants that received no inoculant. Consortium 3 comprises a combination of *Stenotrophomonas maltophilia,* and *Pseudomonas toyotomiensis.* Isolate 10 comprises a pure culture of *Serratia grimesii.* Consortium 4 comprises a combination of *Serratia grimesii* and *Rhodococcus erythropolis.* The increased yields from a microbial inoculant comprising a combination of *Serratia grimesii* and *Rhodococcus erythropolis* is also demonstrated in Figure 4.

In a sixth embodiment of the present invention, the microbial inoculant is a combination of *Rhodococcus erythropolis*, *Pseudomonas toyotomiensis*, and *Stenotrophomonas maltophilia.* In a seventh embodiment of the present invention, the microbial inoculant comprises a combination of *Rhodococcus erythropolis, Serratia grimesii, Stenotrophomonas maltophilia,* and *Pseudomonas toyotomiensis.*

As set out in Figure 4, Consortium 5 comprises a combination of *Rhodococcus erythropolis, Pseudomonas toyotomiensis,* and *Stenotrophomonas maltophilia* and Consortium 7 comprises a combination of *Rhodococcus erythropolis, Serratia grimesii, Stenotrophomonas maltophilia,* and *Pseudomonas toyotomiensis.* Figure 4 shows the average percent yield increases for a hydroponic lettuce inoculant trial in comparison to the control plants that received no inoculant. Consortium 4 comprises a combination of *Serratia grimesii* and *Rhodococcus erythropolis.* Consortium 6 comprises a combination of *Rhodococcus erythropolis* and *Pseudomonas stutzeri.* Isolate 3 comprises a pure culture of *Rhodococcus erythropolis.* Consortium 6 is the only microbial inoculant not to show increased yield.

As shown in Figure 5, the seventh embodiment of the present invention (Consortium 7) which is a combination of *Rhodococcus erythropolis, Serratia grimesii, Stenotrophomonas maltophilia*, and *Pseudomonas toyotomiensis* shows improved weight yields compared to the average fresh lettuce yields for a hydroponic lettuce which received no microbial inoculant, in alignment with the percent yield increase shown for the seventh embodiment in Figure 4. As also shown in Figure 5, improved weight yields over control were also found with an eighth embodiment of the present invention (Isolate 11) which is a pure culture of *Shinella zoogloeoides*, and with a ninth embodiment of the present invention (Consortium 8) which is a combination of *Rhodococcus erythropolis, Serratia grimesii, Stenotrophomonas maltophilia, Pseudomonas toyotomiensis,* and *Shinella zoogloeoides.*

None of the bacterial species of *Rhodococcus erythropolis*, *Stenotrophomonas maltophilia*, *Serratia grimesii*, *Pseudomonas toyotomiensis, and Shinella zoogloeoides* in the microbial inoculants of the present invention were reported in scientific peer reviewed literature as plant growth promoters in hydroponic systems. The present invention is the use of these microbial inoculants comprising *Rhodococcus erythropolis, Stenotrophomonas maltophilia*, *Serratia grimesii Pseudomonas toyotomiensis, and Shinella zoogloeoides* for hydroponic lettuce or other leafy plants, including kale, arugula, and spinach.

To develop the microbial inoculants of the present invention lettuce roots were processed to obtain root associated microbes. The bacteria were purified to single isolates. In total, 105 unique lettuce isolates were obtained. These isolates were not genetically modified.

The bacterial strains were screened for a wide range of biochemical and genetic characteristics that are known to increase agricultural yields. Additionally, bacterial strains were tested against a set of nine antibiotics covering the major antibiotic classes. Bacteria with high levels of multi-drug resistance were excluded from downstream testing.

The 105 bacterial strains underwent whole genome sequencing using Oxford Nanopore MinION technology. Using an in-house bioinformatics pipeline, their genes were analyzed for plant growth promoting capabilities. Bacteria that had both low antibiotic resistance and high levels of favourable genetic traits and biochemical lab results were tested in hydroponics lettuce to measure increases in growth rate.

Of the 105 candidate bacteria strains, only 21 indicated potential to contribute to plant growth promotion. These 21 strains were subsequently tested in a hydroponics grow tent versus control lettuce that was grown without probiotics. Of the 21 promising strains, only 10 resulted in increased lettuce biomass. These 10 strains were mixed into a range of combinations to further increase plant yields.

Extensive grow tent testing on over 8000 plants was required to determine both the individual strains and consortiums that consistently increased crop yields. The host plant specificity required a complexity of consortium formulations. A probiotic mixture that synergistically utilizes the beneficial contributions from several separate plant growth promoters is the result of a year of scientific pursuit. Figures 1 to 5 on lettuce biomass yield data shows a benefit of the present invention.

In use, the microbial inoculants of the present invention are applied to the root zone of the plant either through direct application or by addition to the watering system. In addition to the bacterial cells, the microbial inoculant may also comprise bacterial growth medium and stabilizing agents to increase shelf life.

The trials set out in the figures use the leafy green plant of lettuce, namely *Lactuca sativa,* grown hydroponically. It will be understood that the microbial inoculants may be used on other leafy green plants that are grown hydroponically. Hydroponics growers often have multiple species of leafy green varieties growing concurrently within their farming setup. Examples of other leafy green plants that will receive the positive benefits of the present invention's microbial inoculants are kale, arugula, and spinach.

The microbial inoculants of the present invention were developed to be compatible with use in multiple different hydroponics growing systems and hydroponics substrates (e.g. sponges, coco coir, rockwool, and peat moss). The microbial inoculants of the present invention may be used with leafy green plants in hydroponic based growing systems, aeroponic based growing systems, and other soilless based growing systems, since the microbes are root associated and substrate independent. The microbial inoculant can be applied to whatever medium the leafy green plants are growing in or be added to the water supplying the plants so that the microbial inoculant can contact the roots and begin colonization.

The microbial inoculants of the present invention include probiotics with identical or nearly identical 16S gene sequences as in the sequence listings. Sequence listing 1 is that of *Rhodococcus erythropolis*, sequence listing 2 is that of *Serratia grimesii,* sequence listing 3 is that of *Stenotrophomonas maltophilia*, sequence listing 4 is that of *Pseudomonas toyotomiensis,* and sequnece listing 5 is that of *Shinella zoogloeoides.*

The present invention includes microbial inoculants comprising a probiotic or combination of probiotics identical or nearly identical or 98% identical to the respective sequence listings 1 to 5 of:
*Rhodococcus erythropolis;*
*Rhodococcus erythropolis* and *Serratia grimesii*;
*Serratia grimesii*;
*Serratia grimesii* and *Pseudomonas toyotomiensis*;
*Pseudomonas toyotomiensis* and *Stenotrophomonas maltophilia*;
*Rhodococcus erythropolis*, *Pseudomonas toyotomiensis* and *Stenotrophomonas maltophilia*; *Rhodococcus erythropolis*, *Pseudomonas toyotomiensis*, *Stenotrophomonas maltophilia* and *Serratia grimesii*
*Shinella zoogloeoides*; and
*Rhodococcus erythropolis, Pseudomonas toyotomiensis, Stenotrophomonas maltophilia*, *Serratia grimesii* and *Shinella zoogloeoides.*

Probiotics with similar or corresponding 16S gene sequences, including 98% similarity, may generally be expected to have similar characteristics and similar beneficial effects on leafy green plants. A higher percent identity indicates a closer relationship between two or more microbial strains or isolates. A high degree of similarity or relatedness between two closely related microbial strains or isolates may also indicate a similar ability to impart the same or similar positive agricultural trait or benefit to a leafy green plant. A 16S gene sequence of sequence listing 1 to 5 corresponds to a 16S gene sequence of a second microbial strain if the two stains optimally align.

Probiotic cultures of the microbial inoculants of the present invention may be prepared using any standard or known fermentation techniques, or developed. Those skilled in microbial growth parameters are capable of determining the appropriate nutrients and conditions, including media and incubation conditions. Similarly, the microbial inoculants may comprise various formulations to support the maintenance and stability of the probiotic. Microbial inoculant compositions may comprise a pure or substantially pure population or culture of the microbial strain or isolate, or combination described herein. The term microbial inoculant refers to the combination of microbial strain or isolate combined with one or more other ingredients to form a composition that can be applied to the media or into the water for leafy green plants. The microbial inoculants of the present invention comprise an effective amount of the probiotic or combination of probiotics to impart a positive benefit on a leafy green plant. Compositions of the microbial inoculant include any agricultural carrier or other materials that benefit the probiotic culture and/or benefit or have no adverse effect on the leafy green plant.

While embodiments of the invention have been described in the detailed description, the scope of the claims should not be limited by the preferred embodiments set forth in the examples, but should be given the broadest interpretation consistent with the description as a whole.

### Clauses

1. A microbial inoculant for promoting leafy green plant growth comprising *Rhodococcus erythropolis.*
2. The microbial inoculant of claim 1, wherein the *Rhodococcus erythropolis* has a 98% nucleotide sequence similarity to the 16S region of SEQ ID NO:1.
3. The microbial inoculant of claim 1, also comprising *Serratia grimesii.*
4. A microbial inoculant for promoting leafy green plant growth comprising *Serratia grimesii.*
5. The microbial inoculant of claim 4, wherein the *Serratia grimesii* has a 98% nucleotide sequence similarity to the 16S region of SEQ ID NO:2.
6. The microbial inoculant of claim 5, also comprising *Rhodococcus erythropolis* with a 98% nucleotide sequence similarity to the 16S region of SEQ ID NO:1.
7. The microbial inoculant of claim 4, also comprising *Pseudomonas toyotomiensis.*
8. The microbial inoculant of claim 5, also comprising *Pseudomonas toyotomiensis* with a 98% nucleotide sequence similarity to the 16S region of SEQ ID NO:4.
9. A microbial inoculant for promoting leafy green plant growth comprising *Stenotrophomonas maltophilia* and *Pseudomonas toyotomiensis.*
10. The microbial inoculant of claim 9, wherein the *Stenotrophomonas maltophilia* has a 98% nucleotide sequence similarity to the 16S region of SEQ ID NO:3 and the *Pseudomonas toyotomiensis* has a 98% nucleotide sequence similarity to the 16S region of SEQ ID NO:4.
11. The microbial inoculant of claim 9 also comprising *Rhodococcus erythropolis.*
12. The microbial inoculant of claim 10 also comprising *Rhodococcus erythropolis* with a 98% nucleotide sequence similarity to the 16S region of SEQ ID NO:1.
13. The microbial inoculant of claim 11, also comprising *Serratia grimesii.*
14. The microbial inoculant of claim 12, also comprising *Serratia grimesii* with a 98% nucleotide sequence similarity to the 16S region of SEQ ID NO:2.
15. A microbial inoculant for promoting leafy green plant growth comprising *Shinella zoogloeoides.*
16. The microbial inoculant of claim 15, wherein the *Shinella zoogloeoides* has a 98% nucleotide sequence similarity to the 16S region of SEQ ID NO:5.
17. The microbial inoculant of claim 13, also comprising *Shinella zoogloeoides.*
18. The microbial inoculant of claim 14, also comprising *Shinella zoogloeoides* with a 98% nucleotide sequence similarity to the 16S region of SEQ ID NO:5.
19. The microbial inoculant of any one of claims 1 to 18 in which the leafy green plant is *Lactuca sativa.*
20. The use of the microbial inoculant of any one of claims 1 to 19, in one of the growing systems of hydroponic, aeroponic, soilless or soil based.
21. A method for promoting plant growth comprising applying a composition of the microbial inoculant of any one of claims 1 to 18 directly or through a water supply to the roots of the leafy green plants in an amount that provides for positive benefits relative to a control leafy green plant that does not receive an application of said microbial inoculant.

### SEQUENCE LISTINGS

**SEQ ID NO:1 *Rhodococcus erythropolis*** 
TTTGATCCTGGCTCAGGACGAACGCTGGCGGCGTGCTTAACACATGCAAGTCGAGCGGTA
AGGCCTTTCGGGGTACACGAGCGGCGAACGGGTGAGTAACACGTGGGTGATCTGCCCTGC
ACTTCGGGATAAGCCTGGGAAACTGGGTCTAATACCGGATATGACCTCCTATCGCATGGT
GGGTGGTGGAAAGATTTATCGGTGCAGGATGGGCCCGCGGCCTATCAGCTTGTTGGTGGG
GTAATGGCCTACCAAGGCGACGACGGGTAGCCGACCTGAGAGGGTGACCGGCCACACTG
GGACTGAGACACGGCCCAGACTCCTACGGGAGGCAGCAGTGGGGAATATTGCACAATGG
GCGAAAGCCTGATGCAGCGACGCCGCGTGAGGGATGACGGCCTTCGGGTTGTAAACCTCT
TTCAGCAGGGACGAAGCGCAAGTGACGGTACCTGCAGAAGAAGCACCGGCTAACTACGT
GCCAGCAGCCGCGGTAATACGTAGGGTGCAAGCGTTGTCCGGAATTACTGGGCGTAAAG
AGTTCGTAGGCGGTTTGTCGCGTCGTTTGTGAAAACCAGCAGCTCAACTGCTGGCTTGCA
GGCGATACGGGCAGACTTGAGTACTGCAGGGGAGACTGGAATTCCTGGTGTAGCGGTGA
AATGCGCAGATATCAGGAGGAACACCGGTGGCGAAGGCGGGTCTCTGGGCAGTAACTGA
CGCTGAGGAACGAAAGCGTGGGTAGCGAACAGGATTAGATACCCTGGTAGTCCACGCCG
TAAACGGTGGGCGCTAGGTGTGGGTTCCTTCCACGGAATCCGTGCCGTAGCTAACGCATT
AAGCGCCCCGCCTGGGGAGTACGGCCGCAAGGCTAAAACTCAAAGGAATTGACGGGGGC
CCGCACAAGCGGCGGAGCATGTGGATTAATTCGATGCAACGCGAAGAACCTTACCTGGGT
TTGACATATACCGGAAAGCTGCAGAGATGTGGCCCCCCTTGTGGTCGGTATACAGGTGGT
GCATGGCTGTCGTCAGCTCGTGTCGTGAGATGTTGGGTTAAGTCCCGCAACGAGCGCAAC
CCCTATCTTATGTTGCCAGCACGTTATGGTGGGGACTCGTAAGAGACTGCCGGGGTCAAC
TCGGAGGAAGGTGGGGACGACGTCAAGTCATCATGCCCCTTATGTCCAGGGCTTCACACA
TGCTACAATGGCCAGTACAGAGGGCTGCGAGACCGTGAGGTGGAGCGAATCCCTTAAAG
CTGGTCTCAGTTCGGATCGGGGTCTGCAACTCGACCCCGTGAAGTCGGAGTCGCTAGTAA
TCGCAGATCAGCAACGCTGCGGTGAATACGTTCCCGGGCCTTGTACACACCGCCCGTCAC
GTCATGAAAGTCGGTAACACCCGAAGCCGGTGGCTTAACCCCTTGTGGGAGGGAGCCGTC
GAAGGTGGGATCGGCGATTGGGACGAAGTCGTAACAAGGTAGCCGTACCGGAAGGTGCG
GCTGGATCACCTCC
**SEO ID NO:2 *Serratia grimesii*** 
TTGAAGAGTTTGATCATGGCTCAGATTGAACGCTGGCGGCAGGCCTAACACATGCAAGTC
GAGCGGTAGCACAAGAGAGCTTGCTCTCTGGGTGACGAGCGGCGGACGGGTGAGTAATG
TCTGGGAAACTGCCTGATGGAGGGGGATAACTACTGGAAACGGTAGCTAATACCGCATA
ACGTCTTCGGACCAAAGTGGGGGACCTTCGGGCCTCACGCCATCAGATGTGCCCAGATGG
GATTAGCTAGTAGGTGGGGTAATGGCTCACCTAGGCGACGATCCCTAGCTGGTCTGAGAG
GATGACCAGCCACACTGGAACTGAGACACGGTCCAGACTCCTACGGGAGGCAGCAGTGG
GGAATATTGCACAATGGGCGCAAGCCTGATGCAGCCATGCCGCGTGTGTGAAGAAGGCCT
TCGGGTTGTAAAGCACTTTCAGCGAGGAGGAAGGGTAGTGTGTTAATAGCACATTGCATT
GACGTTACTCGCAGAAGAAGCACCGGCTAACTCCGTGCCAGCAGCCGCGGTAATACGGA
GGGTGCAAGCGTTAATCGGAATTACTGGGCGTAAAGCGCACGCAGGCGGTTTGTTAAGTC
AGATGTGAAATCCCCGCGCTTAACGTGGGAACTGCATTTGAAACTGGCAAGCTAGAGTCT
TGTAGAGGGGGGTAGAATTCCAGGTGTAGCGGTGAAATGCGTAGAGATCTGGAGGAATA
CCGGTGGCGAAGGCGGCCCCCTGGACAAAGACTGACGCTCAGGTGCGAAAGCGTGGGGA
GCAAACAGGATTAGATACCCTGGTAGTCCACGCTGTAAACGATGTCGACTTGGAGGTTGT
GCCCTTGAGGCGTGGCTTCCGGAGCTAACGCGTTAAGTCGACCGCCTGGGGAGTACGGCC
GCAAGGTTAAAACTCAAATGAATTGACGGGGGCCCGCACAAGCGGTGGAGCATGTGGTT
TAATTCGATGCAACGCGAAGAACCTTACCTACTCTTGACATCCAGAGAATTCGCTAGAGA
TAGCTTAGTGCCTTCGGGAACTCTGAGACAGGTGCTGCATGGCTGTCGTCAGCTCGTGTTG
TGAAATGTTGGGTTAAGTCCCGCAACGAGCGCAACCCTTATCCTTTGTTGCCAGCACGTA
ATGGTGGGAACTCAAAGGAGACTGCCGGTGATAAACCGGAGGAAGGTGGGGATGACGTC
AAGTCATCATGGCCCTTACGAGTAGGGCTACACACGTGCTACAATGGCGTATACAAAGAG
AAGCGAACTCGCGAGAGCAAGCGGACCTCATAAAGTACGTCGTAGTCCGGATCGGAGTC
TGCAACTCGACTCCGTGAAGTCGGAATCGCTAGTAATCGTAGATCAGAATGCTACGGTGA
ATACGTTCCCGGGCCTTGTACACACCGCCCGTCACACCATGGGAGTGGGTTGCAAAAGAA
GTAGGTAGCTTAACCTTCGGGAGGGCGCTTACCACTTTGTGATTCATGACTGGGGTGAAG
TCGTAACAAGGTAACCGTAGGGGAACCTGCGGTTGGATCACCTCCT
**SEQ ID NO:3 *Stenotrophomonas maltophilia*** 
TGAAGAGTTTGATCCTGGCTCAGAGTGAACGCTGGCGGTAGGCCTAACACATGCAAGTCG
AACGGCAGCACAGAGGAGCTTGCTCCTTGGGTGGCGAGTGGCGGACGGGTGAGGAATAC
ATCGGAATCTACTTTTTCGTGGGGGATAACGTAGGGAAACTTACGCTAATACCGCATACG
ACCTACGGGTGAAAGCAGGGGATCTTCGGACCTTGCGCGATTGAATGAGCCGATGTCGGA
TTAGCTAGTTGGCGGGGTAAAGGCCCACCAAGGCGACGATCCGTAGCTGGTCTGAGAGG
ATGATCAGCCACACTGGAACTGAGACACGGTCCAGACTCCTACGGGAGGCAGCAGTGGG
GAATATTGGACAATGGGCGCAAGCCTGATCCAGCCATACCGCGTGGGTGAAGAAGGCCTT
CGGGTTGTAAAGCCCTTTTGTTGGGAAAGAAATCCAGCCGGCTAATACCTGGTTGGGATG
ACGGTACCCAAAGAATAAGCACCGGCTAACTTCGTGCCAGCAGCCGCGGTAATACGAAG
GGTGCAAGCGTTACTCGGAATTACTGGGCGTAAAGCGTGCGTAGGTGGTTATTTAAGTCC
GTTGTGAAAGCCCTGGGCTCAACCTGGGAACTGCAGTGGATACTGGATGACTAGAATGTG
GTAGAGGGTAGCGGAATTCCTGGTGTAGCAGTGAAATGCGTAGAGATCAGGAGGAACAT
CCATGGCGAAGGCAGCTACCTGGACCAACATTGACACTGAGGCACGAAAGCGTGGGGAG
CAAACAGGATTAGATACCCTGGTAGTCCACGCCCTAAACGATGCGAACTGGATGTTGGGT
GCAATTTGGCACGCAGTATCGAAGCTAACGCGTTAAGTTCGCCGCCTGGGGAGTACGGTC
GCAAGACTGAAACTCAAAGGAATTGACGGGGGCCCGCACAAGCGGTGGAGTATGTGGTT
TAATTCGATGCAACGCGAAGAACCTTACCTGGCCTTGACATGTCGAGAACTTTCCAGAGA
TGGATGGGTGCCTTCGGGAACTCGAACACAGGTGCTGCATGGCTGTCGTCAGCTCGTGTC
GTGAGATGTTGGGTTAAGTCCCGCAACGAGCGCAACCCTTGTCCTTAGTTGCCAGCACGT
AATGGTGGGAACTCTAAGGAGACCGCCGGTGACAAACCGGAGGAAGGTGGGGATGACGT
CAAGTCATCATGGCCCTTACGGCCAGGGCTACACACGTACTACAATGGTAGGGACAGAGG
GCTGCAAGCCGGCGACGGTAAGCCAATCCCAGAAACCCTATCTCAGTCCGGATTGGAGTC
TGCAACTCGACTCCATGAAGTCGGAATCGCTAGTAATCGCAGATCAGCATTGCTGCGGTG
AATACGTTCCCGGGCCTTGTACACACCGCCCGTCACACCATGGGAGTTTGTTGCACCAGA
AGCAGGTAGCTTAACCTTCGGGAGGGCGCTTGCCACGGTGTGGCCGATGACTGGGGTGAA
GTCGTAACAAGGTAGCCGTATCGGAAGGTGCGGCTGGATCACCTCCTT
**SEQ ID NO:4 *Pseudomonas toyotomiensis*** 
TGAAGAGTTTGATCATGGCTCAGATTGAACGCTGGCGGCAGGCCTAACACATGCAAGTCG
AGCGGATGAGGGGAGCTTGCTCCCTGATTTAGCGGCGGACGGGTGAGTAATGCCTAGGA
ATCTGCCTAGTGGTGGGGGATAACGTTCCGAAAGGAACGCTAATACCGCATACGTCCTAC
GGGAGAAAGCGGGGGATCTTCGGACCTCGCGCCATTAGATGAGCCTAGGTCGGATTAGCT
AGTTGGTGAGGTAAAGGCTCACCAAGGCGACGATCCGTAACTGGTCTGAGAGGATGATC
AGTCACACTGGAACTGAGACACGGTCCAGACTCCTACGGGAGGCAGCAGTGGGGAATAT
TGGACAATGGGCGAAAGCCTGATCCAGCCATGCCGCGTGTGTGAAGAAGGTCTTCGGATT
GTAAAGCACTTTAAGTTGGGAGGAAGGGTATTCACCTAATACGTGAGTATTTTGACGTTA
CCGACAGAATAAGCACCGGCTAACTTCGTGCCAGCAGCCGCGGTAATACGAAGGGTGCA
AGCGTTAATCGGAATTACTGGGCGTAAAGCGCGCGTAGGTGGTTCGTTAAGTTGGATGTG
AAAGCCCCGGGCTCAACCTGGGAACTGCATCCAAAACTGGCGAGCTAGAGTACGGTAGA
GGGTGGTGGAATTTCCTGTGTAGCGGTGAAATGCGTAGATATAGGAAGGAACACCAGTG
GCGAAGGCGACCACCTGGACTGATACTGACACTGAGGTGCGAAAGCGTGGGGAGCAAAC
AGGATTAGATACCCTGGTAGTCCACGCCGTAAACGATGTCAACTAGCCGTTGGAATCCTT
GAGATTTTAGTGGCGCAGCTAACGCATTAAGTTGACCGCCTGGGGAGTACGGCCGCAAGG
TTAAAACTCAAATGAATTGACGGGGGCCCGCACAAGCGGTGGAGCATGTGGTTTAATTCG
AAGCAACGCGAAGAACCTTACCTGGCCTTGACATGCTGAGAACTTTCCAGAGATGGATTG
GTGCCTTCGGGAACTCAGACACAGGTGCTGCATGGCTGTCGTCAGCTCGTGTCGTGAGAT
GTTGGGTTAAGTCCCGTAACGAGCGCAACCCTTGTCCTTAGTTACCAGCACGTTATGGTGG
GCACTCTAAGGAGACTGCCGGTGACAAACCGGAGGAAGGTGGGGATGACGTCAAGTCAT
CATGGCCCTTACGGCCAGGGCTACACACGTGCTACAATGGTCGGTACAAAGGGTTGCCAA
GCCGCGAGGTGGAGCTAATCCCATAAAACCGATCGTAGTCCGGATCGCAGTCTGCAACTC
GACTGCGTGAAGTCGGAATCGCTAGTAATCGTGAATCAGAATGTCACGGTGAATACGTTC
CCGGGCCTTGTACACACCGCCCGTCACACCATGGGAGTGGGTTGCTCCAGAAGTAGCTAG
TCTAACCTTCGGGGGGACGGTTACCACGGAGTGATTCATGACTGGGGTGAAGTCGTAACA
AGGTAGCCGTAGGGGAACCTGCGGCTGGATCACCTCCT
**SEO ID NO:5 *Shinella zoogloeoides***

TTTGATCCTGGCTCAGAACGAACGCTGGCGGCAGGCTTAACACATGCAAGTCGAACGCCC
CGCAAGGGGAGTGGCAGACGGGTGAGTAACGCGTGGGAATCTACCCATCTCTACGGAAT
AACTCAGGGAAACTTGTGCTAATACCGTATACGCCCTTCGGGGGAAAGATTTATCGGAGA
TGGATGAGCCCGCGTTGGATTAGCTAGTTGGTGGGGTAAAGGCCTACCAAGGCGACGATC
CATAGCTGGTCTGAGAGGATGATCAGCCACATTGGGACTGAGACACGGCCCAAACTCCTA
CGGGAGGCAGCAGTGGGGAATATTGGACAATGGGCGCAAGCCTGATCCAGCCATGCCGC
GTGAGTGATGAAGGCCCTAGGGTTGTAAAGCTCTTTCACCGGTGAAGATAATGACGGTAA
CCGGAGAAGAAGCCCCGGCTAACTTCGTGCCAGCAGCCGCGGTAATACGAAGGGGGCTA
GCGTTGTTCGGAATTACTGGGCGTAAAGCGCACGTAGGCGGGTATTTAAGTCAGGGGTGA
AATCCCAGAGCTCAACTCTGGAACTGCCTTTGATACTGGGTACCTAGAGTATGGAAGAGG
TAAGTGGAATTCCGAGTGTAGAGGTGAAATTCGTAGATATTCGGAGGAACACCAGTGGCG
AAGGCGGCTTACTGGTCCATTACTGACGCTGAGGTGCGAAAGCGTGGGGAGCAAACAGG
ATTAGATACCCTGGTAGTCCACGCCGTAAACGATGAATGTTAGCCGTCGGCATGCATGCA
TGTCGGTGGCGCAGCTAACGCATTAAACATTCCGCCTGGGGAGTACGGTCGCAAGATTAA
AACTCAAAGGAATTGACGGGGGCCCGCACAAGCGGTGGAGCATGTGGTTTAATTCGAAG
CAACGCGCAGAACCTTACCAGCCCTTGACATGTCGGTCGCGGATTACAGAGATGTTTTCC
TTCAGTTAGGCTGGACCGAACACAGGTGCTGCATGGCTGTCGTCAGCTCGTGTCGTGAGA
TGTTGGGTTAAGTCCCGCAACGAGCGCAACCCTCGCCCTTAGTTGCCAGCATTCAGTTGG
GCACTCTAAGGGGACTGCCGGTGATAAGCCGAGAGGAAGGTGGGGATGACGTCAAGTCC
TCATGGCCCTTACGGGCTGGGCTACACACGTGCTACAATGGTGGTGACAGTGGGCAGCGA
GACAGCGATGTCGAGCTAATCTCCAAAAGCCATCTCAGTTCGGATTGCACTCTGCAACTC
GAGTGCATGAAGTTGGAATCGCTAGTAATCGCGGATCAGCATGCCGCGGTGAATACGTTC
CCGGGCCTTGTACACACCGCCCGTCACACCATGGGAGTTGGTTTTACCCGAAGGCACTGC
GCTAACCGCAAGGGGGCAGGTGACCACGGTAGGGTCAGCGACTGGGGTGAAGTCGTAAC
AAGGTAGCCGTAGGGGAACCTGCGGCTGGATCACCTCC

## Claims

1. A microbial inoculant for promoting leafy green plant growth comprising the bacterial species:
i) *Rhodococcus erythropoli,* optionally in combination with *Serratia grimesia*,
ii) *Serratia grimesia*, optionally in combination with *Pseudomonas toyotomiensis,*
iii) *Shinella zoogloeoides*, or
iv) *Pseudomonas toyotomiensis* in combination *Stenotrophomonas maltophilia*, and optionally further in combination with and *Rhodococcus erythropoli* and optionally also *Serratia grimesia* and further optionally also in combination with *Shinella zoogloeoides.*

2. The microbial inoculant of claim 1 for promoting leafy green plant growth comprising *Rhodococcus erythropolis,* preferably wherein the *Rhodococcus erythropolis* has a 98% nucleotide sequence similarity to the 16S region of SEQ ID NO: 1.

3. The microbial inoculant of claim 2, also comprising *Serratia grimesii.*

4. The microbial inoculant of claim 1 for promoting leafy green plant growth comprising *Serratia grimesii,* preferably wherein the *Serratia grimesii* has a 98% nucleotide sequence similarity to the 16S region of SEQ ID NO:2.

5. The microbial inoculant of claim 4, also comprising *Rhodococcus erythropolis* with a 98% nucleotide sequence similarity to the 16S region of SEQ ID NO: 1.

6. The microbial inoculant of claim 4, also comprising *Pseudomonas toyotomiensis.*

7. The microbial inoculant of claim 4, also comprising *Pseudomonas toyotomiensis* with a 98% nucleotide sequence similarity to the 16S region of SEQ ID NO:4.

8. The microbial inoculant of claim 1 for promoting leafy green plant growth comprising *Stenotrophomonas maltophilia* and *Pseudomonas toyotomiensis,* preferably wherein the *Stenotrophomonas maltophilia* has a 98% nucleotide sequence similarity to the 16S region of SEQ ID NO:3 and the *Pseudomonas toyotomiensis* has a 98% nucleotide sequence similarity to the 16S region of SEQ ID NO:4.

9. The microbial inoculant of claim 8 also comprising *Rhodococcus erythropolis.*

10. The microbial inoculant of claim 8 also comprising *Rhodococcus erythropolis* with a 98% nucleotide sequence similarity to the 16S region of SEQ ID NO: 1.

11. The microbial inoculant of claim 9, also comprising *Serratia grimesii.*

12. The microbial inoculant of claim 10, also comprising *Serratia grimesii* with a 98% nucleotide sequence similarity to the 16S region of SEQ ID NO:2.

13. The microbial inoculant of claim 1 for promoting leafy green plant growth comprising *Shinella zoogloeoides,* preferably wherein the *Shinella zoogloeoides* has a 98% nucleotide sequence similarity to the 16S region of SEQ ID NO:5.

14. The microbial inoculant of claim 11, also comprising *Shinella zoogloeoides.*

15. The microbial inoculant of claim 12, also comprising *Shinella zoogloeoides* with a 98% nucleotide sequence similarity to the 16S region of SEQ ID NO:5.

16. The microbial inoculant of any one of claims 1 to 15 in which the leafy green plant is *Lactuca sativa.*

17. The use of the microbial inoculant of any one of claims 1 to 16, in one of the growing systems of hydroponic, aeroponic, soilless or soil based.

18. A method for promoting plant growth comprising applying a composition of the microbial inoculant of any one of claims 1 to 16 directly or through a water supply to the roots of the leafy green plants in an amount that provides for positive benefits relative to a control leafy green plant that does not receive an application of said microbial inoculant.
